# EUROPEAN PATENT SPECIFICATION

(11) **EP 4 622 953 B1**
(45) Date of publication and mention of the grant of the patent: **28.10.2026**
(21) Application number: 23802295.8
(22) Date of filing: 13.11.2023
(51) Int. Cl.: C07C 231/02, C07C 253/20, C07C 255/57, C07C 253/30

(54) **PREPARATION OF 4-CYANOBENZOYL CHLORIDES FROM ALKALI METAL 4-CARBAMOYL-BENZOATES**
HERSTELLUNG VON 4-CYANOBENZOYLCHLORIDEN AUS ALKALIMETALL-4-CARBAMOYLBENZOATEN
PRÉPARATION DE CHLORURES DE 4-CYANOBENZOYLE À PARTIR DE CARBAMOYL-BENZOATES DE MÉTAL ALCALIN

(30) Priority: 23.11.2022 EP 22209203
(43) Date of publication of application: 01.10.2025
(73) Proprietor: BASF Agricultural Solutions Deutschland GmbH, 67117 Limburgerhof (DE)
(72) Inventor: GEBHARDT, Joachim, 67056 Ludwigshafen am Rhein (DE); KOENEMANN, Martin, 67056 Ludwigshafen am Rhein (DE); KUTONOVA, Ksenia, 67056 Ludwigshafen am Rhein (DE); EHRESMANN, Manfred, 67056 Ludwigshafen am Rhein (DE); ZEUMKE, Marcus, 67056 Ludwigshafen am Rhein (DE); GOETZ, Roland, 67056 Ludwigshafen am Rhein (DE)
(74) Representative: AP-IP
(86) International application number: PCT/EP2023/081556
(87) International publication number: WO 2024/110227

(56) References cited:
- WO-A1-2021/209377

## Description

The present invention relates to a process for the preparation of 4-cyanobenzoyl chlorides of formula I from alkali metal 4-carbamoyl-benzoate of formula II with a chlorinating agent and a catalyst.

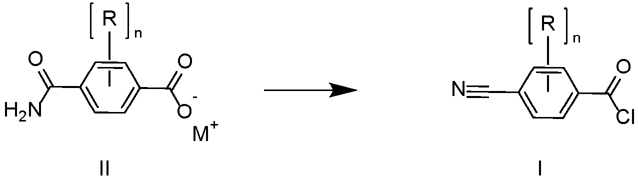

4-Cyanobenzoyl chlorides of formula I are versatile and highly reactive synthetic intermediates offering various strategic options for orthogonal synthesis concepts taking advantage of the differentiated reactivity of the nitrile and the chloro carbonyl group towards nucleophilic reactants. For example, compounds of formula I can be employed in the preparation of benzamide type trifluoromethyl-1,2,4-oxadiazoles, for example, compounds disclosed in WO 2015/185485 A1 and WO 2017/211649 A1. These are potent agrochemicals controlling phytopathogenic fungi.

Synthetic access to 4-cyanobenzoyl chlorides of formula I via 4-carbamoylbenzoic acids of type II is particularly attractive since it taps into industrial feedstock such as terephthalic acid dichloride or corresponding diesters, which are available on large scale and at a low price.

It is part of the common general knowledge in the art of synthetic chemistry that carboxylic acids or salts thereof react with suitable chlorinating agents to yield corresponding carboxylic acid chlorides. Likewise, it is widely known that primary carboxamides undergo dehydration with the same type of chlorinating agents to obtain corresponding nitriles. However, there is little known about selective transformations of compounds featuring both these functional groups in one molecule, and to transform both functional groups in one step, without mutual interference, using one type of chlorinating agent.

This scarcity of reported procedures originates in the skilled person's awareness that carboxylic acid halides are highly reactive electrophiles reacting non-selectively with nucleophilic species, for example with primary carboxamides or other intermediate species that are prone to intermolecular reactions. Given these properties the skilled person would expect a number of side reactions when using chlorinating agents with bifunctional compounds of formula II, including, amongst others, the undesired formation of oligomers and/or polymers.

WO 2021/209377 A1 describes a process for the preparation of 4-cyanobenzoyl chlorides through reaction of 4-carbamoylbenzoic acids with various types of chlorinating agents, in particular with phosphoryl chloride.

In order to improve the overall synthetic efficiency in multistep processes it may be desirable to use alkali metal benzoates of formula II instead of carbamoylbenzoic acids.

The inventors of the present invention found that phosphoryl trichloride in reactions with alkali metal benzoates of formula II do not yield 4-cyanobenzoyl chlorides of formula I (as opposed to reactions using the free acid as demonstrated in WO 2021/209377) but instead lead to the formation of substantive amounts of terephthalic acid dichloride alongside other side-products (see working example 6 herein).

The inventors further found that employing thionyl chloride in these reactions suffers the same disadvantage of poor selectivity and provides low to moderate yields of 4-cyanobenzoyl chlorides (see working example 7 herein).

The inventors surprisingly found that almost quantitative yields of compounds I can be obtained by reaction of alkali metal benzoate of formula II with chlorinating agents selected from the group consisting of phosgene, diphosgene, and triphosgene; the process further comprising an auxiliary solvent and the presence of a carboxamide catalyst. This process is economically viable as it enables the efficient preparation of compounds of formula I on an industrial scale in high yield and essentially avoiding the formation of undesirable side-products, such as terephthalic acid dichloride.

Accordingly, the present invention relates to a process for the preparation of 4-cyanobenzoyl chlorides of formula I, 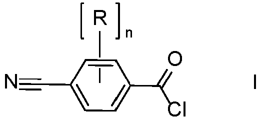 wherein
- R: is halogen, C₁-C₄-alkyl, C₁-C₄-haloalkyl, C₁-C₄-alkoxy, or C₁-C₄-haloalkoxy;
- n: is 0, 1, or 2;

the process comprising reacting an alkali metal 4-carbamoylbenzoate of formula II, 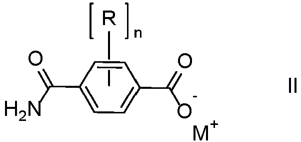
wherein the variables n and R in the alkali metal benzoate of formula II are as defined above for compounds of formula I, and wherein M⁺ is an alkali metal cation,
with a chlorinating agent selected from the group consisting of phosgene, diphosgene, and triphosgene; the process further comprising an auxiliary solvent and the presence of a carboxamide catalyst of formula C in substoichiometric amounts, based on the amount of alkali metal benzoate of formula II, 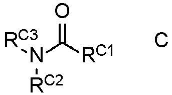
wherein
   - R^{C1}: is hydrogen, methyl, or NR^{C4}R^{C5}; wherein R^{C4} and R^{C5} independently from each other are selected from the group consisting of hydrogen and C₁-C₄-alkyl;
   - R^{C2}: is C₁-C₄-alkyl;
   or
   - R^{C1} and R^{C2}: together with the group -NR^{C3}-C(=O)- to which they are bound form a saturated or partially unsaturated 5- or 6-membered heterocycle, wherein the heterocycle optionally includes, beside one nitrogen atom, 1 or 2 heteroatoms independently selected from N, O and S as ring member atoms with the provision that the heterocycle cannot contain 2 contiguous atoms selected from O and S; and wherein one or two CH₂ groups of the heterocycle may be replaced by one or two groups independently selected from C(=O) and C(=S);
   - R^{C3}: is hydrogen or C₁-C₄-alkyl;
   or
   - R^{C2} and R^{C3}: together with the nitrogen atom to which they are bound form a saturated or partially unsaturated 5- or 6-membered heterocycle, wherein the heterocycle optionally includes, beside one nitrogen atom, 1 or 2 heteroatoms independently selected from N, O and S as ring member atoms with the provision that the heterocycle cannot contain 2 contiguous atoms selected from O and S; and wherein one or two CH₂ groups of the heterocycle may be replaced by one or two groups independently selected from C(=O) and C(=S).

In one aspect of the present invention the alkali metal cation in the alkali metal benzoate of formula II is potassium or sodium, preferably potassium.

In a preferred embodiment the chlorinating agent of the process of the invention is phosgene.

Typically, the amount of the chlorinating agent in the process of the present invention is between 2 and 5 equivalents, preferably between 2 and 3 equivalents, based on the amount of compound II.

The process of the present invention is conducted in an auxiliary solvent. The term "auxiliary solvent" herein refers to an inert aprotic organic solvent, which acts merely as a solvent and is not consumed in the course of the reaction. For the avoidance of doubt an auxiliary solvent is not identical with the reactants such as compounds II, the chlorinating agent, or the catalyst. Suitable auxiliary solvents are, for example, aliphatic, cycloaliphatic and aromatic hydrocarbons (non-limiting examples are: pentane, hexane, heptane, octane, nonane, decane, petroleum ether, cyclohexane, methylcyclohexane, benzene, toluene, xylene), aliphatic halogen-hydrocarbons (non-limiting examples are: methylene chloride, chloroform, di- and tetrachloroethane), nitriles (non-limiting examples are: acetonitrile, propionitrile, benzonitrile), ethers (non-limiting examples are: diethylether, dibutylether, tert-butylmethylether, tetrahydrofuran, ethylene glycol dimethyl ether, ethylene glycol, diethyl ether, diethylene glycol dimethyl ether, dioxane, diethylene, glycol monomethyl- or monoethyl ether), and sulphoxides and sulphones (non-limiting examples are: dimethyl sulfoxide, dimethyl sulfone, tetramethylene sulfoxide, tetramethylene sulfone).

In one aspect of the invention the auxiliary solvents are selected from the group consisting of nitriles, ethers, sulphoxides, sulphones, aliphatic, cycloaliphatic and aromatic hydrocarbons bearing 1 to 10 carbon atoms, and whereas these hydrocarbons are halogenated or non-halogenated; or mixtures thereof.

Preferred auxiliary solvents are selected from the group consisting of dioxane, *tert*-butyl methyl ether, di-*iso*-propyl ether, tetrahydrofuran, benzene, toluene, xylene, mesitylene, chlorobenzene, *n*-hexane, *n*-heptane, *n*-octane, *n*-nonane, *n*-decane, cyclohexane, dichloromethane, chloroform, tetrachloromethane, dichloroethane; or mixtures thereof.

The process of the present invention requires the presence of substoichiometric amounts of a carboxamide catalyst of formula C, based on the amount of alkali metal benzoate of formula II.

In one embodiment the catalyst of formula C is used in substoichiometric amounts, based on the amount of alkali metal benzoate of formula II, 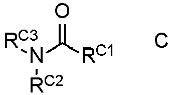 wherein
- R^{C1}: is hydrogen or methyl;
- R^{C2}: is C₁-C₄-alkyl;
- R^{C3}: is hydrogen or C₁-C₄-alkyl.

In one aspect of the present invention the process is conducted in the presence of substoichiometric amounts, based on the amount of compound II, of *N*,*N*-dimethylformamide, *N*,*N*-diethylformamide, *N,N*-di-*n*-propylformamide, *N*,*N*-diisopropylformamide, *N,N*-di-*n-*butylformamide, *N*,*N*-diisobutylformamide, *N,N*-di-*tert*-butylformamide *N*,*N*-dimethylacetamide, *N*,*N*-diethylacetamide, *N*,*N*-di-n-propylacetamide, *N*,*N*-diisopropylacetamide, *N,N*-di*-n-*butylacetamide, *N*,*N*-diisobutylacetamide, *N,N*-di-*tert*-butylacetamide, *N*-methylformamide, *N-*ethylformamide, *N*-*n*-propylformamide, *N*-isopropylformamide, *N*-*n*-butylformamide, *N-*isobutylformamide, *N*-*tert*-butylformamide, *N*-methylacetamide, *N*-ethylacetamide, *N*-*n-*propylacetamide, *N*-isopropylacetamide, *N*-*n*-butylacetamide, *N*-isobutylacetamide, *N-tert-*butylacetamide, *N*-acetylpyrrolidine. 1-acetyl-2-pyrrolidone, acetylpiperidine, 1-acetyl-2-piperidinone, urea, *N,N'*-dimethylurea, tetramethylurea, 2-imidazolidinone, 1,3-dimethyl-2-imidazolidinone, tetrahydro-2(1*H*)-pyrimidinone, 1,3-dimethyl-3,4,5,6-tetrahydro-2(1*H*)-pyrimidinone; preferably *N,N*-dimethylformamide and *N,N*-dimethylacetamide.

In one aspect the catalyst C is used in an amount of up to 0.5 equivalents, up to 0.2 equivalents, or up to 0.1 equivalents, based on the amount of compound II.

In another aspect the catalyst C is used in an amount that is in the range between 0.001 and 0.1 equivalents, or between 0.01 and 0.1 equivalents, based on the amount of compound II.

In another aspect *N*,*N*-dimethylformamide or *N*,*N*-dimethylacetamide, preferably *N*,*N-*dimethylformamide, is used in an amount of up to 0.5 equivalents, up to 0.2 equivalents, or up to 0.1 equivalents, based on the amount of compound II. In another aspect *N*,*N-*dimethylformamide or *N*,*N*-dimethylacetamide, preferably *N*,*N*-dimethylformamide, is used in an amount that is in the range between 0.001 and 0.1 equivalents, or between 0.01 and 0.1 equivalents, based on the amount of compound II.

The inventors found that the temperature in the process of the present invention can be varied in a wide range. The reaction may be conducted at ambient temperature or the reaction mixture may be heated to reflux. Theoretical considerations as well as experimental results suggest that lower temperature results in lower conversion rates, i.e. longer reaction times. Accordingly, the reaction proceeded faster at a higher temperature but the inventors also detected higher amounts of unwanted by-products formed at elevated temperature, especially at reflux conditions. In one aspect the process of the present invention is conducted at a temperature in the range of from 20 °C to reflux temperature, or at a temperature in the range of from 20 °C to 90 °C, or at a temperature in the range of from 20 °C to 70 °C; preferably at a temperature in the range of from 40 °C to 90 °C, or in the range of from 40 °C to 70 °C; most preferably in the range of from 50 °C to 80 °C.

In a particularly preferred embodiment the process of the present invention is conducted in benzene, toluene, xylene, mesitylene, chlorobenzene, *n*-hexane, *n*-heptane, *n*-octane, *n-*nonane, *n*-decane, cyclohexane, or mixtures thereof, and the reaction mixture is heated in the range of from 50 °C to 80 °C.

In a further particularly preferred embodiment the process of the present invention is conducted in toluene and the reaction mixture is heated to a temperature in the range of from 50 °C to 80 °C.

The reaction is carried out at pressures within a range between 100 kPa (1 bar) and 500 kPa, preferably between 100 kPa and 300 kPa. Most preferably, the reaction is carried out at atmospheric pressure.

The reaction is generally carried out within 1 to 12 hours; preferably within 1 to 8 hours; more preferably within 1 to 6 hours.

In one aspect of the present invention the variable n is 0 or 1 and R is fluorine in compounds of formulae I or II.

In a preferred embodiment the variable n is 0 in compounds of formulae I or II.

In a preferred embodiment (embodiment E.1) of the present invention the alkali metal cation in the alkali metal benzoate of formula II is potassium or sodium.

Embodiment E.2: is based on embodiment E.1, wherein the chlorinating agent is phosgene. Embodiment E.3: is based on embodiment E.2, wherein the auxiliary solvent is dioxane, *tert-*butyl methyl ether, di-*iso*-propyl ether, tetrahydrofuran, benzene, toluene, xylene, mesitylene, chlorobenzene, *n*-hexane, *n*-heptane, *n*-octane, *n*-nonane, *n*-decane, cyclohexane, dichloromethane, chloroform, tetrachloromethane, dichloroethane, or mixtures thereof. Embodiment E.4: is based on embodiment E.3, wherein the reaction is conducted in the presence of *N,N*-dimethylformamide or *N,N*-dimethylacetamide in an amount of up to 0.5 equivalents, based on the amount of alkali metal benzoate of formula II.

Embodiment E.5: is based on embodiment E.4, wherein the reaction mixture is heated to a temperature in the range of from 20 °C to reflux temperature.

Embodiment E.6: is based on embodiment E.4, wherein the reaction mixture is heated to a temperature in the range of from 40 °C to 90 °C.

Embodiment E.7: is based on embodiment E.4, wherein the reaction mixture is heated to a temperature in the range of from 50 °C to 80 °C.

Embodiment E.8: is based on embodiment E.5, wherein the pressure is within a range between 100 kPa and 500 kPa.

Embodiment E.9: is based on embodiment E.6, wherein the pressure is within a range between 100 kPa and 500 kPa.

Embodiment E.10: is based on embodiment E.7, wherein the pressure is within a range between 100 kPa and 500 kPa.

Embodiment E.11: is based on embodiment E.5, wherein the reaction is carried out at atmospheric pressure.

Embodiment E.12: is based on embodiment E.6, wherein the reaction is carried out at atmospheric pressure.

Embodiment E.13: is based on embodiment E.7, wherein the reaction is carried out at atmospheric pressure.

Embodiment E.14: is based on any one of embodiments E.1 to E.13, wherein n is 0 or 1 and R is fluorine in compounds of formulae I or II.

Embodiment E.15: is based on any one of embodiments E.1 to E.13, wherein n is 0 in compounds of formulae I or II.

Compounds of formula II can be obtained in a two-step process involving mono-saponification of terephthalic acid diester of formula IIa, 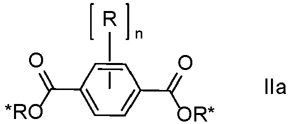
wherein R* is C₁-C₈-alkyl, wherein the alkyl group is unsubstituted or substituted with 1 or 2 hydroxy groups, with an alkali metal hydroxide, such as sodium hydroxide or potassium hydroxide (described in Industrial Engineering Chemistry 1957, 49, 10, 1692), to give a compound of formula IIb, 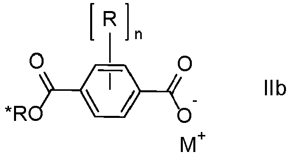
wherein M⁺ is the corresponding alkali metal cation. In a second step compound IIb is reacted with ammonia, for example as described in DE 2313580, to give a compound of formula II, whereas the variables n and R in compounds Ila and Ilb are as defined for compounds of formulae I and II herein. The diesters of formula IIa are either commercially available or they can be prepared from commercially available starting materials using synthetic procedures that are well known to the skilled person in the art.

In a further embodiment the present invention relates to a process comprising the step of reacting the compound of formula I, wherein the variable n is 0, with an amine of formula III,

R¹-NH-R² III

wherein
- R¹: is C₁-C₆-alkyl, C₁-C₆-alkoxy, C₃-C₁₁-cycloalkyl, C₃-C₈-cycloalkenyl, C₂-C₆-alkenyl, C₂-C₆-alkynyl, C₁-C₆-alkoxyimino-C₁-C₄-alkyl, C₂-C₆-alkenyloxyimino-C₁-C₄-alkyl, C₂-C₆-alkynyloxyimino-C₁-C₄-alkyl, C₁-C₆-alkylamino, diC₁-C₆-alkylamino, -C(=O)-C₁-C₆-alkyl, -C(=O)-O-C₁-C₆-alkyl, phenyl-C₁-C₄-alkyl, phenyl-C₁-C₄-alkenyl, phenyl-C₁-C₄-alkynyl, heteroaryl-C₁-C₄-alkyl, phenyl, naphthyl, or a 3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms with the provision that the heterocycle cannot contain 2 contiguous atoms selected from O and S; and wherein the heteroaryl group in the group heteroaryl-C₁-C₄-alkyl is a 5- or 6-membered aromatic heterocycle, wherein the ring member atoms of the heterocyclic ring include besides carbon atoms 1, 2, 3 or 4 heteroatoms selected from N, O, and S as ring member atoms with the provision that the heterocycle cannot contain 2 contiguous atoms selected from O and S; and wherein any of the above-mentioned aliphatic or cyclic groups are unsubstituted or substituted with 1, 2, 3, or up to the maximum possible number of identical or different groups R^{1a}; or
- R¹ and R²,: together with the nitrogen atom to which they are attached, form a saturated or partially unsaturated mono- or bicyclic 3- to 10-membered heterocycle, wherein the heterocycle includes beside one nitrogen atom and one or more carbon atoms no further heteroatoms or 1, 2 or 3 further heteroatoms independently selected from N, O, and S as ring member atoms with the provision that the heterocycle cannot contain 2 contiguous atoms selected from O and S; and wherein the heterocycle is unsubstituted or substituted with 1, 2, 3, 4, or up to the maximum possible number of identical or different groups R^{1a}; wherein
- R^{1a}: is halogen, oxo, cyano, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, -NHSO₂-C₁-C₄-alkyl, -(C=O)-C₁-C₄-alkyl, C(=O)-C₁-C₄-alkyl, C₁-C₆-alkylsulfonyl, hydroxyC₁-C₄-alkyl, -C(=O)-NH₂, -C(=O)-NH(C₁-C₄-alkyl), C₁-C₄-alkylthio-C₁-C₄-alkyl, aminoC₁-C₄-alkyl, C₁-C₄-alkylamino-C₁-C₄-alkyl, diC₁-C₄-alkylamino-C₁-C₄-alkyl, aminocarbonyl-C₁-C₄-alkyl, or C₁-C₄-alkoxy-C₁-C₄-alkyl;
- R²: is hydrogen, C₁-C₆-alkyl, C₂-C₆-alkenyl, C₂-C₆-alkynyl, C₁-C₆-alkoxy, C₃-C₁₁-cycloalkyl, -C(=O)H, -C(=O)-C₁-C₆-alkyl, -C(=O)-C₃-C₁₁-cycloalkyl, or -C(=O)-O-C₁-C₆-alkyl; and wherein any of the aliphatic or cyclic groups in R² are unsubstituted or substituted with 1, 2, 3, or up to the maximum possible number of identical or different radicals selected from the group consisting of halogen, hydroxy, oxo, cyano, C₁-C₆-alkyl, C₁-C₆-alkoxy, and C₃-C₁₁-cycloalkyl;
to obtain a compound of formula IV

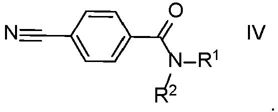

Analogous transformations are described in WO 2013/008162 A1, WO 2015/185485 A1, or WO 2017/211652 A1 and the references cited therein.

The amines of formula III are either commercially available or can be prepared, for example, according to R. C. Larock, Comprehensive Organic Transformations, Verlag Wiley-VCH, 2nd Edition 1999, pages 1929 ff..

In a further embodiment the present invention relates to a process comprising the step of reacting the compound of formula IV with hydroxylamine or its hydrochloride salt, in the presence of a base, preferably triethylamine, sodium hydroxide or sodium methylate, in a suitable solvent, such as methanol, ethanol or water, or a mixture of these solvents, at a temperature between 0 °C and 100 °C to obtain a compound of formula Va, 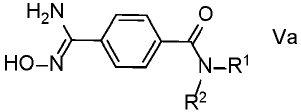 which is further reacted with an activated derivative of trifluoroacetic acid, for example ethyl trifluoroacetate, trifluoroacetic anhydride or trifluoroacetic chloride, to obtain a compound of formula V

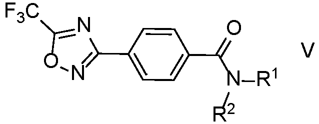

For related examples see Kitamura, S. et al., Chem. Pharm. Bull. 2001, 49, 268 or WO 2013/008162 A1 or WO 2015/185485 A1.

In another embodiment, the compound of formula V is reacted with a suitable thionylating reagent to obtain a compound of formula VI, 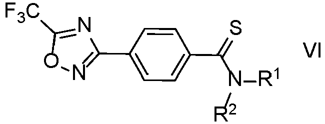 as described in WO 2019/020451 A1 and WO 2017/211649 A1 and the references cited therein.

In a preferred embodiment the variables R¹ and R² in compounds of formula III, IV, V and VI have the following meaning:
- R¹: is methyl, ethyl, *n*-propyl, *iso*-propyl, *n*-butyl, *sec*-butyl, *iso*-butyl, cyclopropyl, 2-methoxyiminoethyl, bicyclo[1.1.1]pentan-1-yl, or phenyl; and wherein the phenyl group is unsubstituted or substituted with 1, 2, 3 or up to the maximum possible number of identical or different radicals selected from the group consisting of fluorine, chlorine, cyano, OH, NH₂, methyl, ethyl, methoxy, trifluoromethyl, trifluoromethoxy, difluoromethyl, difluoromethoxy, and cyclopropyl; and
- R²: is hydrogen, methyl, or ethyl.

In another preferred embodiment the variables R¹ and R² in compounds of formula III, IV, V and VI have the following meaning:
- R¹: is methyl, 2-methoxyiminoethyl, bicyclo[1.1.1]pentan-1-yl, 2-fluoro-phenyl, 4-fluoro-phenyl, or 2,4-difluorophenyl; in particular methyl or 2-fluoro-phenyl; and
- R²: is hydrogen.

In the definitions of the variables given above, collective terms are used which are generally representative for the substituents in question.

The term "Cₙ-Cₘ" indicates the number of carbon atoms possible in each case in the substituent or substituent moiety in question.

The term "halogen" refers to fluorine, chlorine, bromine and iodine.

The term "oxo" refers to an oxygen atom =O, which is bound to a carbon atom or sulfur atom, thus forming, for example, a ketonyl -C(=O)- or sulfinyl -S(=O)- group.

The term "C₁-Cₙ-alkyl" refers to a straight-chained or branched saturated hydrocarbon group having 1 to n carbon atoms, for example methyl, ethyl, propyl, 1-methylethyl, butyl, 1-methylpropyl, 2-methylpropyl, and 1,1-dimethylethyl.

The term "C₂-C₆-alkenyl" refers to a straight-chain or branched unsaturated hydrocarbon radical having 2 to 6 carbon atoms and a double bond in any position, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₆-alkynyl" refers to a straight-chain or branched unsaturated hydrocarbon radical having 2 to 6 carbon atoms and containing at least one triple bond, such as ethynyl, 1-propynyl, 2-propynyl (propargyl), 1-butynyl, 2-butynyl, 3-butynyl, 1-methyl-2-propynyl.

The term "C₁-C₆-haloalkyl" refers to a straight-chained or branched alkyl group having 1 to 6 carbon atoms (as defined above), wherein some or all of the hydrogen atoms in these groups may be replaced by halogen atoms as mentioned above, for example chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, CH₂-C₂F₅, CF₂-C₂F₅, CF(CF₃)₂, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl.

The term "C₁-C₆-alkoxy" refers to a straight-chain or branched alkyl group having 1 to 6 carbon atoms (as defined above) which is bonded via an oxygen, at any position in the alkyl group, for example methoxy, ethoxy, *n*-propoxy, 1-methylethoxy, butoxy, 1-methylpropoxy, 2-methylpropoxy or 1,1-dimethylethoxy.

The term "C₁-C₆-haloalkoxy" refers to a C₁-C₆-alkoxy group as defined above, wherein some or all of the hydrogen atoms may be replaced by halogen atoms as mentioned above, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, OC₂F₅, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy, 1-(CH₂Br)-2-bromoethoxy, 4-fluorobutoxy, 4-chlorobutoxy, 4-bromobutoxy or nonafluorobutoxy.

The terms "phenyl-C₁-C₄-alkyl or heteroaryl-C₁-C₄-alkyl" refer to alkyl having 1 to 4 carbon atoms (as defined above), wherein one hydrogen atom of the alkyl radical is replaced by a phenyl or hetereoaryl radical respectively.

The term "C₁-C₄-alkoxy-C₁-C₄-alkyl" refers to alkyl having 1 to 4 carbon atoms (as defined above), wherein one hydrogen atom of the alkyl radical is replaced by a C₁-C₄-alkoxy group (as defined above). Likewise, the term "C₁-C₄-alkylthio-C₁-C₄-alkyl" refers to alkyl having 1 to 4 carbon atoms (as defined above), wherein one hydrogen atom of the alkyl radical is replaced by a C₁-C₄-alkylthio group.

The term "C₁-C₆-alkylthio" as used herein refers to straight-chain or branched alkyl groups having 1 to 6 carbon atoms (as defined above) bonded via a sulfur atom. Accordingly, the term "C₁-C₆-haloalkylthio" as used herein refers to straight-chain or branched haloalkyl group having 1 to 6 carbon atoms (as defined above) bonded through a sulfur atom, at any position in the haloalkyl group.

The term "C₁-C₄-alkoxyimino" refers to a divalent imino radical (C₁-C₄-alkyl-O-N=) carrying one C₁-C₄-alkoxy group as substituent, e.g. methylimino, ethylimino, propylimino, 1-methylethyl-imino, butylimino, 1-methylpropylimino, 2-methylpropylimino, 1,1-dimethylethylimino and the like.

The term "C₁-C₆-alkoxyimino-C₁-C₄-alkyl" refers to alkyl having 1 to 4 carbon atoms, wherein two hydrogen atoms of one carbon atom of the alkyl radical are replaced by a divalent C₁-C₆-alkoxyimino radical (C₁-C₆-alkyl-O-N=) as defined above.

The term "C₂-C₆-alkenyloxyimino-C₁-C₄-alkyl" refers to alkyl having 1 to 4 carbon atoms, wherein two hydrogen atoms of one carbon atom of the alkyl radical are replaced by a divalent C₂-C₆-alkenyloxyimino radical (C₂-C₆-alkenyl-O-N=).

The term "C₂-C₆-alkynyloxyimino-C₁-C₄-alkyl" refers to alkyl having 1 to 4 carbon atoms, wherein two hydrogen atoms of one carbon atom of the alkyl radical are replaced by a divalent C₂-C₆-alkynyloxyimino radical (C₂-C₆-alkynyl-O-N=).

The term "hydroxyC₁-C₄-alkyl" refers to alkyl having 1 to 4 carbon atoms, wherein one hydrogen atom of the alkyl radical is replaced by a OH group.

The term "aminoC₁-C₄-alkyl" refers to alkyl having 1 to 4 carbon atoms, wherein one hydrogen atom of the alkyl radical is replaced by a NH₂ group.

The term "C₁-C₆-alkylamino" refers to an amino group, which is substituted with one residue independently selected from the group that is defined by the term C₁-C₆-alkyl. Likewise, the term "diC₁-C₆-alkylamino" refers to an amino group, which is substituted with two residues independently selected from the group that is defined by the term C₁-C₆-alkyl.

The term "C₁-C₄-alkylamino-C₁-C₄-alkyl" refers to refers to alkyl having 1 to 4 carbon atoms (as defined above), wherein one hydrogen atom of the alkyl radical is replaced by a C₁-C₄-alkyl-NH-group which is bound through the nitrogen. Likewise, the term "diC₁-C₄-alkylamino-C₁-C₄-alkyl" refers to refers to alkyl having 1 to 4 carbon atoms (as defined above), wherein one hydrogen atom of the alkyl radical is replaced by a (C₁-C₄-alkyl)₂N- group which is bound through the nitrogen.

The term "aminocarbonyl-C₁-C₄-alkyl" refers to alkyl having 1 to 4 carbon atoms, wherein one hydrogen atom of the alkyl radical is replaced by a -(C=O)-NH₂ group.

The term "C₃-C₁₁-cycloalkyl" refers to a monocyclic, bicyclic or tricyclic saturated univalent hydrocarbon radical having 3 to 11 carbon ring members that is connected through one of the ring carbon atoms by substitution of one hydrogen atom, such as cyclopropyl (C₃H₅), cyclobutyl, cyclopentyl, cyclohexyl, cycloheptyl, cyclooctyl, bicyclo[1.1.0]butyl, bicyclo[2.1.0]pentyl, bicyclo[1.1.1]pentyl, bicyclo[3.1.0]hexyl, bicyclo[2.1.1]hexyl, norcaranyl (bicyclo[4.1.0]heptyl) and norbornyl (bicyclo[2.2.1]heptyl).

The terms "-C(=O)-C₁-C₆-alkyl", "-C(=O)-O-C₁-C₆-alkyl" and "-C(=O)-C₃-C₁₁-cycloalkyl" refer to aliphatic radicals which are attached through the carbon atom of the -C(=O)- group.

The term "aliphatic" refers to compounds or radicals composed of carbon and hydrogen and which are non-aromatic compounds. An "alicyclic" compound or radical is an organic compound that is both aliphatic and cyclic. They contain one or more all-carbon rings which may be either saturated or unsaturated, but do not have aromatic character.

The terms "cyclic moiety" or "cyclic group" refer to a radical which is an alicyclic ring or an aromatic ring, such as, for example, phenyl or heteroaryl.

The term "and wherein any of the aliphatic or cyclic groups are unsubstituted or substituted with..." refers to aliphatic groups, cyclic groups and groups, which contain an aliphatic and a cyclic moiety in one group, such as in, for example, C₃-C₈-cycloalkyl-C₁-C₄-alkyl; therefore a group which contains an aliphatic and a cyclic moiety both of these moieties may be substituted or unsubstituted independently of each other.

The term "phenyl" refers to an aromatic ring systems incuding six carbon atoms (commonly referred to as benzene ring.

The term "heteroaryl" refers to aromatic monocyclic or polycyclic ring systems incuding besides carbon atoms, 1, 2, 3 or 4 heteroatoms independently selected from the group consisting of N, O and S.

The term "saturated 3- to 7-membered carbocycle" is to be understood as meaning monocyclic saturated carbocycles having 3, 4 or 5 carbon ring members. Examples include cyclopropyl, cyclopentyl, cyclohexyl, cycloheptyl, and the like.

The term "3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms", is to be understood as meaning both, aromatic mono- and bicyclic heteroaromatic ring systems, and also saturated and partially unsaturated heterocycles, for example:
a 3- or 4-membered saturated heterocycle which contains 1 or 2 heteroatoms from the group consisting of N, O and S as ring members such as oxirane, aziridine, thiirane, oxetane, azetidine, thiethane, [1,2]dioxetane, [1,2]dithietane, [1,2]diazetidine;
and a 5- or 6-membered saturated or partially unsaturated heterocycle which contains 1, 2 or 3 heteroatoms from the group consisting of N, O and S as ring members such as 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl and also the corresponding -ylidene radicals; and
a 7-membered saturated or partially unsaturated heterocycle such as tetra- and hexahydroazepinyl, such as 2,3,4,5-tetrahydro[1H]azepin-1-,-2-,-3-,-4-,-5-,-6- or-7-yl, 3,4,5,6-tetrahydro[2H]azepin-2-,-3-,-4-,-5-,-6- or-7-yl, 2,3,4,7-tetrahydro[1H]azepin-1-,-2-,-3-,-4-,-5-,-6- or-7-yl, 2,3,6,7-tetrahydro[1H]azepin-1-,-2-,-3-,-4-,-5-,-6- or-7-yl, hexahydroazepin-1-,-2-,-3- or-4-yl, tetra- and hexahydrooxepinyl such as 2,3,4,5-tetrahydro[1H]oxepin-2-,-3-,-4-,-5-,-6- or-7-yl, 2,3,4,7-tetrahydro[1H]oxepin-2-,-3-,-4-,-5-,-6- or-7-yl, 2,3,6,7-tetrahydro[1H]oxepin-2-, -3-,-4-,-5-,-6- or-7-yl, hexahydroazepin-1-,-2-,-3- or-4-yl, tetra- and hexahydro-1,3-diazepinyl, tetra- and hexahydro-1,4-diazepinyl, tetra- and hexahydro-1,3-oxazepinyl, tetra- and hexahydro-1,4-oxazepinyl, tetra- and hexahydro-1,3-dioxepinyl, tetra- and hexahydro-1,4-dioxepinyl and the corresponding -ylidene radicals.

The term "5- or 6-membered heteroaryl" or the term "5- or 6-membered aromatic heterocycle" refer to aromatic ring systems incuding besides carbon atoms, 1, 2, 3 or 4 heteroatoms independently selected from the group consisting of N, O and S, for example, a 5-membered heteroaryl such as pyrrol-1-yl, pyrrol-2-yl, pyrrol-3-yl, thien-2-yl, thien-3-yl, furan-2-yl, furan-3-yl, pyrazol-1-yl, pyrazol-3-yl, pyrazol-4-yl, pyrazol-5-yl, imidazol-1-yl, imidazol-2-yl, imidazol-4-yl, imidazol-5-yl, oxazol-2-yl, oxazol-4-yl, oxazol-5-yl, isoxazol-3-yl, isoxazol-4-yl, isoxazol-5-yl, thiazol-2-yl, thiazol-4-yl, thiazol-5-yl, isothiazol-3-yl, isothiazol-4-yl, isothiazol-5-yl, 1,2,4-triazolyl-1-yl, 1,2,4-triazol-3-yl 1,2,4-triazol-5-yl, 1,2,4-oxadiazol-3-yl, 1,2,4-oxadiazol-5-yl and 1,2,4-thiadiazol-3-yl, 1,2,4-thiadiazol-5-yl; or a 6-membered heteroaryl, such as pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl and 1,3,5-triazin-2-yl and 1,2,4-triazin-3-yl.

### Working Examples

The present invention is further illustrated by means of the following working examples.

### Analytical Methods

### Quantitative UHPLC method, used for examples 1-2

Column: Xselect HSS PFP 100*2,1mm, 1,8µm, flow: 0.5 mL/min, time: 7 min, temperature: 45 °C; wavelength 240 nm; injector volume: 1 uL; retention time of the respective products is based on reference material. Eluent: A: Water with 0,1 vol% HCOOH; B: methanol with 0,1 vol% HCOOH.

| *Time (min)* | *%B* | *Rate (mL*/*min)* |
|---|---|---|
| *0,0* | *15* | *0.5* |
| *3,0* | *95* | *0.5* |
| *5,0* | *95* | *0.5* |
| *5,1* | *15* | *0.5* |

### Qualitative HPLC method, used for examples 3-7

Column: Agilent Zorbax Extend C18, 4,6*50mm , 1,8 µm, flow: 1,2 mL/min, time: 11 min, pressure: 400 bar; temperature: 25 °C; wavelength 240 nm; injector volume: 1 uL; retention time of the respective products is based on reference material. Eluent: A: Water with 0.1 vol% H₃PO₄; B: Acetonitrile with 0.1 vol% H₃PO₄.

| *Time (min)* | *%B* | *Rate (mL*/*min)* |
|---|---|---|
| *8,0* | *95* | *1,2* |
| *10,0* | *90* | *1,2* |
| *10,1* | *100* | *1,2* |
| *10.9* | *100* | *1,2* |
| *11.0* | *5* | *1,2* |

Sample preparation: all acid chloride samples were converted into the corresponding methyl esters before analysis: Samples were quenched with an excess of methanol over 30 minutes.

Quantitative GC method with internal standard n-decane, used for example 4

| | | | |
|---|---|---|---|
| Column | CP-Sil 5 CB 50m*0,25mm*0,25µm | | |
| Injector temperature | 280 °C | | |
| FID temperature | 320 °C | | |
| Oven temperature | Rate [°C/min] | Value [°C] | Hold Time [min] |
| | - | 60 | 0 |
| | 15 | 300 | 10 |

| Carrier gas | hydrogen | | |
|---|---|---|---|
| Detector | FID | | |
| Split ratio | 100:1 | | |
| Column flow | 1.0 mL/minute (constant flow) | | |
| Injection volume | 1 µL | | |
| Analysis time | 26.0 min | | |

### Example 1: Preparation of potassium methyl terephthalate

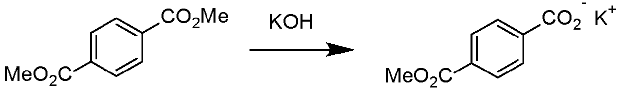

180 g (0.927 mol) Dimethyl terephthalate was charged to a mixture of 489.4 g methanol and 593.3 g toluene. The mixture was heated under agitation to 50 °C. Then 67.3 g KOH (85 %, 1.02 mol) was added in 5 portions over 45 minutes. After stirring the reaction mixture over 4 hours at 50 °C, the reaction vessel was cooled to 20 °C and the precipitated product filtered off. The solid on the nutsch was washed with 250 g methanol and dried over night at 95 °C / 30 mbar in a drying cabinet. 199 g of the title product were obtained with a purity of 97.7 % according to quantitative HPLC analytics, equal to a yield of 96.1 %.

### Example 2: Preparation of potassium 4-carbamoylbenzoate

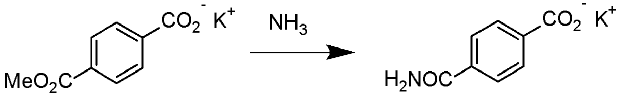

A pressure reactor was charged with 12 g (0.054 mol) potassium monomethyl terephthalate and 109 g mother liquor from the last batch of the same reaction (formamide solvent has been used instead for the first batch) and 12 g fresh formamide. The reactor was purged 3 times with nitrogen at 5 bar, pressurized with 27.8 g (1.632 mol) gaseous ammonia and heated to 110 °C over 7 hours. During the reaction the pressure inside the reactor increased to 18 bar. After completion of the reaction the reaction mixture was cooled down to 30 °C and depressurized. The reactor was purged 3 times with 5 bar of nitrogen and the mixture transferred to another reactor for work-up. After cooling of the suspension to 5 °C over 3 hours under agitation followed by filtration and washing of the solid product with 10 g methanol wet potassium 4-carbamoylbenzoate was obtained, which was dried at 65 °C / 50 mbar in a drying cabinet overnight to give 10.5 g of the title product with a purity of 97.8 % according to quantitative HPLC, equal to a yield of 94.1 %.

The first batch using pure solvent formamide instead of recycled mother liquor gave a significantly lower yield since the batch is used to saturate the solvent with the product. Afterwards more than 15 recycles of mother liquor were performed with similar yields and purities. Methanol did not further accumulate in the mother liquor under the used work up conditions due to its low boiling point.

### Example 3: Preparation of 4-cyanobenzoyl chloride with phosgene

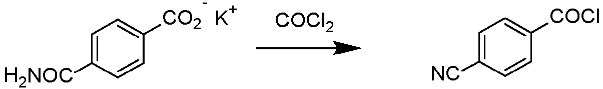

0.6 g (0.0029 mol) Potassium 4-carbamoylbenzoate, 4.8 g (0.0073 mol) of phosgene in toluene (15 %) and 0.022 g (0.0003 mol) N,N-dimethylformamide were charged to 12.3 g toluene in a round-bottomed flask. The mixture was heated to 90 °C under agitation and held at 90 °C over 3 hours. HPLC reaction control revealed complete conversion of the starting material and 95.9 area% of the desired product.

### Example 4: Preparation of 4-cyanobenzoyl chloride with triphosgene

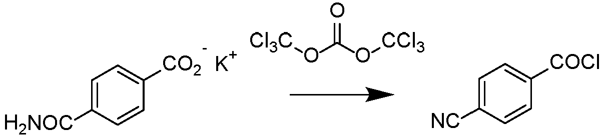

7.1 g (97 %, 0.0339 mol) Potassium 4-carbamoylbenzoate, 10.2 g (0.0339 mol) of triphosgene and 0.025 g (0.0003 mol) *N,N*-dimethylformamide were added to 94.6 g toluene in a round-bottomed flask. The mixture was heated to 70 °C under agitation and held at 70 °C over 7 hours. HPLC reaction control revealed complete conversion of the starting material and 94.2 area% of the desired product. The mixture was cooled to room temperature and the precipitated potassium chloride was filtered off and washed. Toluene was partly evaporated from the combined filtrates under vacuum (85 °C / 15 mbar) and a sample for quantitative analysis of the product via GC was taken indicating 92.6 % crude yield in solution. After evaporation of the remaining toluene, 16.7 g cyclohexane was added. The mixture was heated to reflux and cooled within 3 hours to 23 "C. The product was filtered off, washed twice with each 5 g cyclohexane and finally dried under vacuum (60 °C / 15 mbar), 4.4 g of the title product was obtained (purity 96.1 % according to quantitative HPLC, corresponding to an isolated yield of 80.9 %).

### Example 5: Preparation of 4-cyanobenzoyl chloride with triphosgene followed by conversion with 2-fluoroaniline

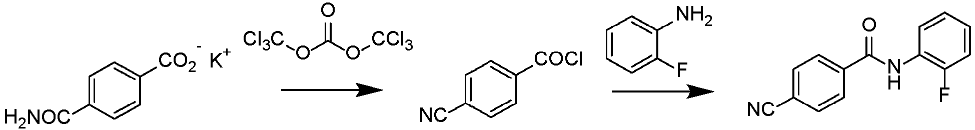

0.2 g (0.001 mol) Potassium 4-carbamoylbenzoate, 0.3 g (0.001 mol) of triphosgene and 0.007 g (0.0001 mol) *N,N*-dimethylformamide was added to 4.1 g toluene in a round-bottomed flask. The mixture was heated to 90 °C under agitation and held at 90 °C for 4.5 hours. HPLC reaction control revealed complete conversion of the starting material and 96.8 area% of the desired product. 0.12 g (0.00108 mol) 2-fluoroaniline was added and the mixture heated to reflux over 5 hours. HPLC revealed full conversion of the 4-cyanobenzoyl chloride to the desired 4-cyano-N-(2-fluorophenyl)-benzamide

### Example 6: (example not according to the present invention) Preparation of 4-cyanobenzoyl chloride with phosphoryl trichloride

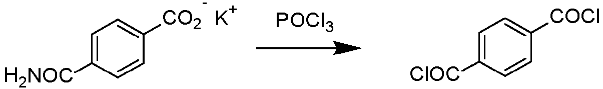

4 g (0.0195 mol) Potassium 4-carbamoylbenzoate and 21.1 g (0.1364 mol) phosphoryl trichloride were charged to a round-bottomed flask. The mixture was heated to 80 °C under agitation and held at 80 °C over 2 hours. HPLC reaction control revealed complete conversion of the starting material but no formation of the desired product. Formation of terephthaloyl dichloride was observed instead (93.0 area-% of the dimethyl terephthalate have been analyzed after quench with methanol).

### Example 7: (example not according to the present invention) Preparation of 4-cyanobenzoyl chloride with thionyl chloride

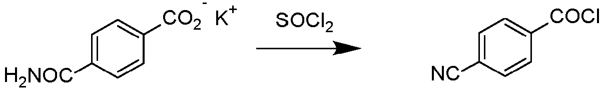

1.5 g (0.0073 mol) Potassium 4-carbamoylbenzoate, 2.2 g (0.0183 mol) of thionyl chloride and 0.005 g (0.0001 mol) *N,N*-dimethylformamide was added to 27 g toluene in a round-bottomed flask. The mixture was heated to 90 °C under agitation and held at 90 °C over 5 hours. HPLC reaction control revealed complete conversion of the starting material but only 65.2 area% of the desired product. 13.3 area% of terephthaloyl chloride (structure see example 7) were generated together with other by-products (structures not elucidated).

## Claims

1. A process for the preparation of 4-cyanobenzoyl chlorides of formula I, 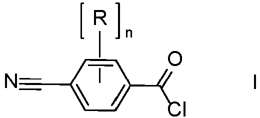 wherein
R is halogen, C₁-C₄-alkyl, C₁-C₄-haloalkyl, C₁-C₄-alkoxy, or C₁-C₄-haloalkoxy;
n is 0, 1, or 2;
the process comprising reacting an alkali metal 4-carbamoylbenzoate of formula II, 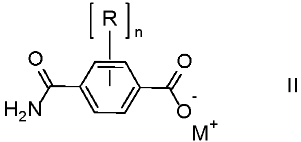
wherein the variables n and R in the alkali metal benzoate of formula II are as defined above for compounds of formula I, and wherein M⁺ is an alkali metal cation,
with a chlorinating agent selected from the group consisting of phosgene, diphosgene, and triphosgene; the process further comprising an auxiliary solvent and the presence of a carboxamide catalyst of formula C in substoichiometric amounts, based on the amount of alkali metal benzoate of formula II, 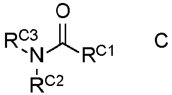
wherein
R^{C1} is hydrogen, methyl, or NR^{C4}R^{C5}; wherein R^{C4} and R^{C5} independently from each other are selected from the group consisting of hydrogen and C₁-C₄-alkyl;
R^{C2} is C₁-C₄-alkyl;
or
R^{C1} and R^{C2} together with the group -NR^{C3}-C(=O)- to which they are bound form a saturated or partially unsaturated 5- or 6-membered heterocycle, wherein the heterocycle optionally includes, beside one nitrogen atom, 1 or 2 heteroatoms independently selected from N, O and S as ring member atoms with the provision that the heterocycle cannot contain 2 contiguous atoms selected from O and S; and wherein one or two CH₂ groups of the heterocycle may be replaced by one or two groups independently selected from C(=O) and C(=S);
R^{C3} is hydrogen or C₁-C₄-alkyl;
or
R^{C2} and R^{C3} together with the nitrogen atom to which they are bound form a saturated or partially unsaturated 5- or 6-membered heterocycle, wherein the heterocycle optionally includes, beside one nitrogen atom, 1 or 2 heteroatoms independently selected from N, O and S as ring member atoms with the provision that the heterocycle cannot contain 2 contiguous atoms selected from O and S; and wherein one or two CH₂ groups of the heterocycle may be replaced by one or two groups independently selected from C(=O) and C(=S).

2. The process according to claim 1, wherein the alkali metal cation in the alkali metal benzoate of formula II is potassium or sodium.

3. The process according to claim 1 or 2, wherein the chlorinating agent is phosgene.

4. The process according to any one of claims 1 to 3, wherein the amount of the chlorinating agent is between 2 and 5 equivalents, based on the amount of the alkali metal benzoate of formula II.

5. The process according to any one of claims 1 to 4, wherein the auxiliary solvents are selected from the group consisting of nitriles, ethers, sulphoxides, sulphones, aliphatic, cycloaliphatic and aromatic hydrocarbons bearing 1 to 10 carbon atoms, and whereas these hydrocarbons are halogenated or non-halogenated; or mixtures thereof.

6. The process according to any one of claims 1 to 4, wherein the auxiliary solvent is selected from the group consisting of dioxane, *tert*-butyl methyl ether, di-*iso*-propyl ether, tetrahydrofuran, benzene, toluene, xylene, mesitylene, chlorobenzene, *n*-hexane, *n-*heptane, *n*-octane, *n*-nonane, *n*-decane, cyclohexane, dichloromethane, chloroform, tetrachloromethane, dichloroethane; or mixtures thereof.

7. The process according to any one of claims 1 to 6, wherein the reaction is conducted in the presence of substoichiometric amounts of *N,N*-dimethylformamide or *N,N-*dimethylacetamide, based on the amount of alkali metal benzoate of formula II.

8. The process according to any one of claims 1 to 7, wherein the temperature is in the range of from 20 °C to reflux temperature.

9. The process according to any one of claims 1 to 8, wherein the temperature is in the range of from 50 °C to 80 °C.

10. The process according to any one of claims 1 to 9, wherein the variable n is 0 or 1 and R in compounds of formulae I and II is fluorine.

11. The process according to any one of claims 1 to 9, wherein the variable n is 0 in compounds of formulae I and II.

12. The process according to any one of claims 1 to 11, further comprising reacting the compound of formula IIb, wherein R* is C₁-C₈-alkyl, wherein the alkyl group is unsubstituted or substituted with 1 or 2 hydroxy groups, and M⁺ is an alkali metal cation, 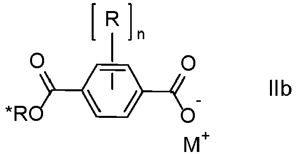 with ammonia to give 4-carbamoylbenzoate of formula II.

13. The process according to claim 12, further comprising reacting the compound of formula IIa, 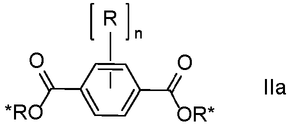 with an alkali metal hydroxide to give a compound of formula Ilb.

14. The process according to any one of claims 11 to 13, further comprising the step of reacting the compound of formula I with an amine of formula III,
R¹-NH-R² III
wherein
R¹ is C₁-C₆-alkyl, C₁-C₆-alkoxy, C₃-C₁₁-cycloalkyl, C₃-C₈-cycloalkenyl, C₂-C₆-alkenyl, C₂-C₆-alkynyl, C₁-C₆-alkoxyimino-C₁-C₄-alkyl, C₂-C₆-alkenyloxyimino-C₁-C₄-alkyl, C₂-C₆-alkynyloxyimino-C₁-C₄-alkyl, C₁-C₆-alkylamino, diC₁-C₆-alkylamino, -C(=O)-C₁-C₆-alkyl, -C(=O)-O-C₁-C₆-alkyl, phenyl-C₁-C₄-alkyl, phenyl-C₁-C₄-alkenyl, phenyl-C₁-C₄-alkynyl, heteroaryl-C₁-C₄-alkyl, phenyl, naphthyl, or a 3- to 10-membered saturated, partially unsaturated or aromatic mono- or bicyclic heterocycle, wherein the ring member atoms of said mono- or bicyclic heterocycle include besides carbon atoms further 1, 2, 3 or 4 heteroatoms selected from N, O and S as ring member atoms with the provision that the heterocycle cannot contain 2 contiguous atoms selected from O and S; and wherein the heteroaryl group in the group heteroaryl-C₁-C₄-alkyl is a 5- or 6-membered aromatic heterocycle, wherein the ring member atoms of the heterocyclic ring include besides carbon atoms 1, 2, 3 or 4 heteroatoms selected from N, O, and S as ring member atoms with the provision that the heterocycle cannot contain 2 contiguous atoms selected from O and S; and wherein any of the above-mentioned aliphatic or cyclic groups are unsubstituted or substituted with 1, 2, 3, or up to the maximum possible number of identical or different groups R^{1a}; or
R¹ and R², together with the nitrogen atom to which they are attached, form a saturated or partially unsaturated mono- or bicyclic 3- to 10-membered heterocycle, wherein the heterocycle includes beside one nitrogen atom and one or more carbon atoms no further heteroatoms or 1, 2 or 3 further heteroatoms independently selected from N, O, and S as ring member atoms with the provision that the heterocycle cannot contain 2 contiguous atoms selected from O and S; and wherein the heterocycle is unsubstituted or substituted with 1, 2, 3, 4, or up to the maximum possible number of identical or different groups R^{1a}; wherein
R^{1a} is halogen, oxo, cyano, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, -NHSO₂-C₁-C₄-alkyl, -(C=O)-C₁-C₄-alkyl, C(=O)-C₁-C₄-alkyl, C₁-C₆-alkylsulfonyl, hydroxyC₁-C₄-alkyl, -C(=O)-NH₂, -C(=O)-NH(C₁-C₄-alkyl), C₁-C₄-alkylthio-C₁-C₄-alkyl, aminoC₁-C₄-alkyl, C₁-C₄-alkylamino-C₁-C₄-alkyl, diC₁-C₄-alkylamino-C₁-C₄-alkyl, aminocarbonyl-C₁-C₄-alkyl, or C₁-C₄-alkoxy-C₁-C₄-alkyl;
R² is hydrogen, C₁-C₆-alkyl, C₂-C₆-alkenyl, C₂-C₆-alkynyl, C₁-C₆-alkoxy, C₃-C₁₁-cycloalkyl, -C(=O)H, -C(=O)-C₁-C₆-alkyl, -C(=O)-C₃-C₁₁-cycloalkyl, or -C(=O)-O-C₁-C₆-alkyl; and wherein any of the aliphatic or cyclic groups in R² are unsubstituted or substituted with 1, 2, 3, or up to the maximum possible number of identical or different radicals selected from the group consisting of halogen, hydroxy, oxo, cyano, C₁-C₆-alkyl, C₁-C₆-alkoxy, and C₃-C₁₁-cycloalkyl;
to obtain a compound of formula IV

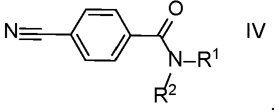

15. The process according to claim 14, further comprising reacting the compound of formula IV to obtain a compound of formula V

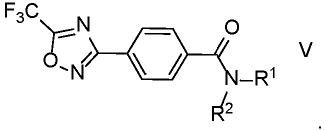

16. The process according to claim 15, further comprising the step of reacting the compound of formula V to obtain a compound of formula VI

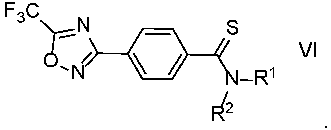

17. The process according to any one of claims 14 to 16, wherein in compounds of formula III, IV, V and VI
R¹ is methyl or 2-fluoro-phenyl; and
R² is hydrogen.

## Patentansprüche

1. Ein Verfahren zur Herstellung von 4-Cyanobenzoylchloriden der Formel I, 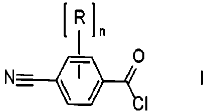 wobei
R is halogen, C₁-C₄-alkyl, C₁-C₄-haloalkyl, C₁-C₄-alkoxy, or C₁-C₄-haloalkoxy;
n ist 0, 1 oder 2;
der Prozess besteht aus der Reaktion eines Alkalimetalls 4-Carbamoylbenzoats der Formel II, 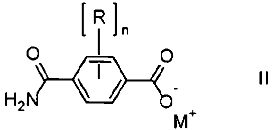
wobei die Variablen n und R im Alkalimetallbenzoat der Formel II sind wie oben für Verbindungen der Formel I definiert, und wobei M+ ein Alkalimetallkation ist,
mit einem chlorierenden Mittel, das aus der Gruppe besteht, bestehend aus Phosgen, Diphosgen, und Triphosgen; der Prozess besteht außerdem aus einem Hilfslösungsmittel und dem Vorhandensein eines Carboxamid-Katalysators der Formel C in substöchiometrischen Mengen, abhängig von der Menge von Alkalimetallbenzoat der Formel II, 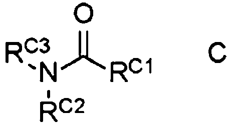
wobei
R^{c1} ist Wasserstoff, Methyl oder NR^{C4}R^{c5};; wobei R^{C4} und R^{C5} unabhängig voneinander sind werden aus der Gruppe ausgewählt, die Wasserstoff und C₁-C₄-Alkyl umfasst;
R^{C2} ist C₁-C₄-Alkyl;
oder
R^{c1} und R^{c2} bilden zusammen mit der Gruppe - NR^{c3}-C(=O)-, an die sie gebunden sind, einen gesättigten oder teilweise ungesättigten 5- oder 6-teiligen Heterozyklus, wobei der Heterozyklus optional neben einem Stickstoffatom 1 oder 2 Heteroatome umfasst unabhängig ausgewählt aus N, 0- und S as Ringelementatomen mit der Bedingung, dass der Heterozyklus nicht zwei zusammenhängende Atome aus O und S enthalten darf; und wobei ein oder zwei CH₂-Gruppen des Heterozyklus durch eine oder zwei unabhängig ausgewählte Gruppen aus C(=O) und C(=S) ersetzt werden können;
R^{c3} ist Wasserstoff oder C₁-C₄-Alkyl;
Oder
R^{c2}und R^{c3} bilden zusammen mit dem Stickstoffatom, an das sie gebunden sind, einen gesättigten oder teilweise ungesättigten 5- oder 6-teiligen Heterozyklus, wobei der Heterozyklus optional neben einem Stickstoffatom 1 oder 2 unabhängig ausgewählte Heteroatome aus N, 0 und Sas-Ringelementatomen umfasst, mit der Voraussetzung, dass Heterozyklus kann nicht zwei zusammenhängende Atome enthalten, die aus O und S ausgewählt werden; und in dem ein oder zwei CH₂-Gruppen des Heterozyklus können durch eine oder zwei unabhängig ausgewählte Gruppen aus C(=O) und C(=S) ersetzt werden.

2. Der Prozess gemäß Anspruch 1, bei dem das Alkalimetall-Kation im Alkalimetall verwendet wird Benzoat der Formel II ist Kalium oder Natrium.

3. Der Prozess gemäß Anspruch 1 oder 2, bei dem das Chlorierungsmittel Phosgen ist.

4. Der Prozess entspricht einem der Ansprüche 1 bis 3, wobei die Menge des Chlorierens. Der Wirkstoff liegt zwischen 2 und 5 Äquivalenten, basierend auf der Menge des Alkalimetallbenzoats von Formel II.

5. Der Prozess gemäß einem der Ansprüche 1 bis 4, bei dem die Hilfslösungsmittel aus der Gruppe ausgewählt werden, die aus Nitrilen, Ethern, Sulphoxiden, Sulphonen und Aliphaten besteht Cycloaliphatische und aromatische Kohlenwasserstoffe mit 1 bis 10 Kohlenstoffatomen, und während diese Kohlenwasserstoffe sind halogeniert oder nicht halogeniert; oder Mischungen davon.

6. Der Prozess gemäß einem der Ansprüche 1 bis 4, bei dem das Hilfslösungsmittel aus der Gruppe aus Dioxan, Tert-Butylmethylether, Di-Iso-Propyläther ausgewählt wird, Tetrahydrofuran, Benzol, Toluol, Xylol, Mesitylen, Chlorbenzol, N-Hexan, *n-*heptan, N-Oktan, N-Nonan, N-Dekan, Cyclohexan, Dichlormethan, Chloroform, Tetrachlorethan, Dichlorethan; oder Mischungen davon.

7. Der Prozess gemäß einem der Ansprüche 1 bis 6, bei dem die Reaktion durchgeführt wird in das Vorhandensein substöchiometrischer Mengen von N,N-Dimethylformamid oder *N,N-*Dimethylacetamid, basierend auf der Menge des Alkalimetallbenzoats der Formel II.

8. Der Prozess entspricht einem der Ansprüche 1 bis 7, wobei die Temperatur im Bereich von 20 °C bis zur Refluxtemperatur liegt.

9. Der Prozess entspricht einem der Ansprüche 1 bis 8, wobei die Temperatur im Bereich von 50 °C bis 80 °C liegt.

10. Der Prozess gemäß einem der Ansprüche 1 bis 9, wobei die Variable n 0 oder 1 ist und R in Verbindungen der Formeln I und II ist Fluor.

11. Der Prozess gemäß einem der Ansprüche 1 bis 9, wobei die Variable n in Zusammensetzungen der Formeln I und II 0 ist .

12. Der Prozess gemäß einem der Ansprüche 1 bis 11 umfasst zudem die Reaktion auf die Zusammensetzung der Formel LLB, wobei R* is C₁-C₈-alkyl ist, wobei die Alkylgruppe unsubstituiert ist oder durch 1 oder 2 Hydroxygruppen ersetzt wird, und M+ ist ein Alkalimetall-Kation, 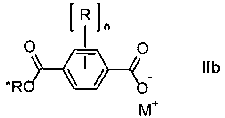 mit Ammoniak, um 4-Carbamoylbenzoat aus Formel II zu erhalten.

13. Der Prozess gemäß Anspruch 12 umfasst außerdem die Reaktion der Verbindung der Formel IIa, 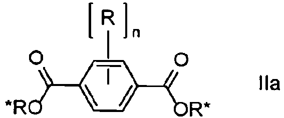 mit einem Alkalimetallhydroxid, um eine Verbindung der Formel LLB zu erhalten.

14. Der Prozess gemäß einem der Ansprüche 11 bis 13 umfasst den Schritt von wobei die Verbindung der Formel I mit einem Amin der Formel III reagiert,
R¹-NH-R² III Krank
wobei
R¹ C₁-C₆-Alkyl,C₁-C₆-Alkoxy, C₃-C₁₁-Cycloalkyl, C₃-C₈-Cycloalkenyl, C₂-C₆-Alkenyl, C₂-C₆-Alkynyl, C₁-C₆-Alkoxyimino- C₁-C₄-Alkyl, C₂-C₆-Alkenyloxyimino- C₁-C₄-al Kyl, C₂-C₆-alkynyloxyimino- C₁-C₄-alkyl, C₁-C₆-alkylamino, di C₁-C₆-alkylamino, -C(=O)- C₁-C₆-alkyl, -C(=O)-O- C₁-C₆-alkyl, Phenyl- C₁-C₄-alkyl, Phenyl- C₁-C₄-alkenyl, Phenyl- C₁-C₄-alkynyl, heteroaryl- C₁-C₄-alkyl, Phenyl, Naphthyl oder ein 3- bis 10-gliedrig gesättigt, teilweise ungesättigt oder aromatisch, mono- oder bicyclisches Heterozyklus, wobei die Ringelementatome des genannten mono- oder bicyclischen Heterozyklus neben Kohlenstoffatomen weitere 1, 2, 3 oder 4 Heteroatome aus N enthalten, 0 und Sas-Ringelementatome mit der Bedingung, dass der Heterozyklus nicht zwei zusammenhängende Atome aus O und S enthalten darf; und wobei die heteroaryle Gruppe in der Gruppe heteroaryl-C₁-C₄-Alkyl ist ein 5- oder 6-leimiger aromatischer Heterozyklus, bei dem die Ringelementatome des heterozyklischen Rings neben Kohlenstoffatomen 1, 2, 3 oder 4 Heteroatome aus N enthalten, 0, und Sas-Ringelementatome mit der Bedingung, dass der Heterozyklus nicht zwei zusammenhängende Atome aus O und S enthalten darf; und wobei eine der oben genannten aliphatischen oder zyklischen Gruppen unersetzt ist oder ersetzt durch 1, 2, 3 oder bis zur maximal möglichen Anzahl identischer oder unterschiedlicher Gruppen R1a; oder
R¹ und R² bilden zusammen mit dem Stickstoffatom, an das sie gebunden sind, einen gesättigten oder teilweise ungesättigten mono- oder bicyclischen 3- bis 10-teiligen Heterozyklus, bei dem der Heterozyklus neben einem Stickstoffatom und einem oder mehreren Kohlenstoffatomen keine weitere Heteroatome oder 1, 2 oder 3 weitere Heteroatome, die unabhängig aus N ausgewählt wurden, 0, und S als Ringelementatome mit der Voraussetzung, dass der Heterozyklus nicht 2 zusammenhängende Atome aus O und S enthalten kann; und wobei der Heterozyklus ist
nicht ersetzt oder durch 1, 2, 3, 4 oder bis zur maximal möglichen Anzahl identischer oder unterschiedlicher Gruppen R1a; wobei
R^{1a} ist Halogen, Oxo, Cyano, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, -NHSO₂- C₁-C₆-alkyl, -(C=O)- C₁-C₄-alkyl, C(=O)- C₁-C₄-alkyl, C₁-C₆-alkylsulfonyl, hydroxy C₁-C₄-alkyl, -C(=O)-NH₂, -C(=O)-NH(C₁-C₄-alkyl), C₁-C₄-alkylthio-C₁-C₄-alkyl, amino C₁-C₄-alkyl, C₁-C₄ alkylamino- C₁-C₄-alkyl, di C₁-C₄-Alkylamino- C₁-C₄-Alkyl, Aminocarbonyl- C₁-C₄-Alkyl oder C₁-C₄-Alkoxy- C₁-C₄-Alkyl;
R² ist Wasserstoff, C₁-C₆-Alkyl, C₂-C₆-Alkenyl, C₂-C₆-Alkynyl, C₁-C₆-Alkoxy, C₃-C₁₁cycloalkyl, -C(=O)H, -C(=O)- C₁-C₆-alkyl, -C(=O)- C₃-C₁₁cycloalkyl oder -C(=O)-O- C1-C6-Alkyl; und wobei eine der aliphatischen oder zyklischen Gruppen in R² unsubstituiert oder durch 1, 2, 3 oder bis zur maximal möglichen Anzahl identischer oder unterschiedlicher Radikale ausgewählt wird, die aus der Gruppe aus Halogen, Hydroxy, Oxo, Cyano, C₁-C₆-Alkyl, C₁-C₆-Alkoxy und C₃-C₁₁cycloalkyl ausgewählt werden;
um eine Verbindung der Formel IV zu erhalten

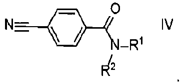

15. Der Prozess gemäß Anspruch 14 umfasst außerdem das Reagieren der Verbindung der Formel IV, um eine Verbindung der Formel V zu erhalten

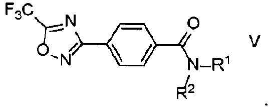

16. Der Prozess gemäß Anspruch 15 umfasst weiter den Schritt, die Verbindung der Formel V zu reagieren, um eine Verbindung der Formel VI zu erhalten

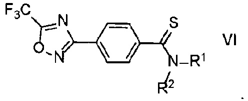

17. Der Prozess gemäß einem der Ansprüche 14 bis 16, wobei in Verbindungen der Formel Ill, IV, Vand VI
R¹ ist Methyl- oder 2-Fluor-Phenyl; und
R² Wasserstoff ist.

## Revendications

1. Un procédé de préparation de 4-chlorures de cyanobenzoyle de formule I, 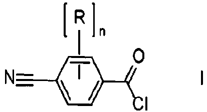 où
R is halogen, C₁-C₄-alkyl, C₁-C₄-haloalkyl, C₁-C₄-alkoxy, or C₁-C₄-haloalkoxy;
n est 0, 1 ou 2 ;
le procédé consistant à réagir un métaux alcali 4-carbamoylbenzoate de formule II, 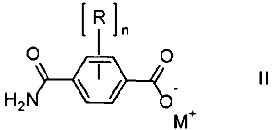
où les variables n et R dans le benzoate de métal alcalin de formule Il sont comme défini ci-dessus pour les composés de formule I, et où M+ est un cation de métal alcalin, avec un agent chlorant sélectionné parmi le groupe composé de phosgène, diphosgène, et triphosgène ; le procédé comprend en outre un solvant auxiliaire et la présence d'un catalyseur de carboxamide de formule C en quantités substœchiométriques, basées sur la quantité de benzoate de métal alcalin de formule II, 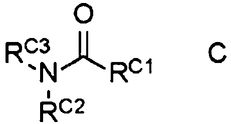 où
R^{c1} est l'hydrogène, le méthyle ou NR^{C4}R^{c5} ; où R^{C4} et R^{C5} sont indépendamment l'un de l'autre
sont sélectionnés parmi le groupe composé d'hydrogène et de C1-C4-alkyle ;
R^{c2} est C₁-C₄-alkyle
; ou
R^{c1} et R^{c2} - avec le groupe - NR^{c3}-C (=O) - auquel ils sont liés, forment un hétérocycle saturé ou partiellement insaturé à 5 ou 6 membres, où l'hétérocycle inclut éventuellement, à côté d'un atome d'azote, 1 ou 2 hétéroatomes sélectionné indépendamment parmi les atomes membres de l'anneau N, 0 et S as à condition que l'hétérocycle ne puisse pas contenir 2 atomes contigus choisis parmi O et S ; et où un ou deux groupes CH₂ de l'hétérocycle peuvent être remplacés par un ou
deux groupes sélectionnés indépendamment parmi C(=O) et C(=S) ;
R^{c3} est de l'hydrogène ou C₁-C₄-alkyle ;
Ou
R^{c2} et R^{c3} , avec l'atome d'azote auquel ils sont liés, forment un hétérocycle saturé ou partiellement insaturé à 5 ou 6 membres, dans lequel l'hétérocycle inclut éventuellement, en plus d'un atome d'azote, 1 ou 2 hétéroatomes sélectionnés indépendamment parmi les atomes membres de l'anneau de N, 0 et Sas, à condition que l'hétérocycle ne peut pas contenir 2 atomes contigus sélectionnés parmi O et S ; et dans lequel un ou deux groupes CH2 de l'hétérocycle peuvent être remplacés par un ou deux groupes sélectionnés indépendamment de C(=O) et C(=S).

2. Le procédé selon la revendication 1, dans lequel le cation du métal alcalin est le métal alcalin Le benzoate de la formule II est le potassium ou le sodium.

3. Le procédé selon la revendication 1 ou 2, dans lequel l'agent chlorant est le phosgène.

4. Le processus selon l'une des revendications 1 à 3, selon laquelle la quantité de chloration agent est entre 2 et 5 équivalents, selon la quantité de benzoate de métal alcalin de Formule II.

5. Le procédé selon l'une des revendications 1 à 4, dans lequel les solvants auxiliaires sont sélectionnés parmi le groupe composé de nitriles, d'éthers, de sulfures, de sulfones, d'aliphatiques,des hydrocarbures cycloaliphatiques et aromatiques contenant de 1 à 10 atomes de carbone, et que ces Les hydrocarbures sont halogénés ou non halogénés ; ou des mélanges de ces mélanges.

6. Le procédé selon l'une des revendications 1 à 4, dans lequel le solvant auxiliaire est sélectionné parmi le groupe constitué du dioxane, du tert-butyl éther méthylique, du di-iso-propyl éther,
Tétrahydrofurane, benzène, toluène, xylène, mésitylène, chlorobenzène, n-hexane, *n-*Heptane, N-octane, N-nonane, N-décane, cyclohexane, dichlorométhane, chloroforme, tétrachlorométhane, dichloroéthane ; ou des mélanges de ces mélanges.

7. Le processus selon l'une des revendications 1 à 6, où la réaction est effectuée dans la présence de quantités substœchiométriques de N,N-diméthylformamide ou *N,N-*diméthylacétamide, basé sur la quantité de benzoate de métal alcalin de la formule II.

8. Le procédé selon l'une des revendications 1 à 7, où la température varie de 20 °C à la température de reflux.

9. Le procédé, selon l'une des revendications 1 à 8, est dans lequel la température se situe entre 50 °C et 80 °C.

10. Le processus selon l'une des revendications 1 à 9, où la variable n est 0 ou 1 et R
dans les composés des formules I et II, il y a du fluor.

11. Le processus selon l'une des revendications 1 à 9, où la variable n est 0 dans les composés des formules I et II.

12. Le processus selon l'une des revendications 1 à 11, comprenant en outre la réaction à la composé de formule IIb, où R* est C 1-C8-alkyle, où le groupe alkyle est non substitué ou remplacé par 1 ou 2 groupes hydroxy, et M+ est un cation de métal alcalin, 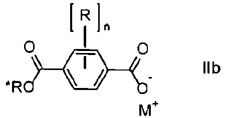 avec de l'ammoniac pour donner le 4-carbamoylbenzoate de la formule II.

13. Le procédé, selon la revendication 12, comprend en outre la réaction du composé de formule IIa, 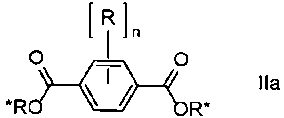 avec un hydroxyde de métal alcalin pour obtenir un composé de formule IIb.

14. Le processus selon l'une des revendications 11 à 13, comprenant en outre l'étape de réagissant le composé de la formule I avec une amine de la formule III,
R¹-NH-R² III Malade
où
R¹ est C₁-C₆-alkyle, C₁-C₆-alkoxy, C₃-C₁₁-cycloalkyle, C₃-C₈-cycloalkényl, C₂-C₆-alkynyle, C₂-C₆-alkynyle, C₁-C₆-alkoxyimino- C₁-C₄-alkyle, C₂-C₆-alkenyloxyimino- C₁-C₄-al kyl, C₂-C₆-alkynyloxyimino- C₁-C₄-alkyl, C₁-C₆-alkylamino, di C₁-C₆-alkylamino, -C(=O)- C₁-C₆-alkyle, -C(=O)-O- C₁-C₆-alkyle, phényl- C₁-C₄-alkyle, phényl- C₁-C₄-alkyl, phényl- C₁-C₄-alkynyl, hétéroaryle- C₁-C₄-alkyle, phényle, naphtyle, ou un alkyle à 3 à 10 membres hétérocycle mono- ou bicyclique saturé, partiellement insaturé ou aromatique, où les atomes membres de l'anneau de cet hétérocycle mono- ou bicyclique incluent, en plus des atomes de carbone, 1, 2, 3 ou 4 hétéroatomes sélectionnés parmi N, 0 et atomes membres de l'anneau Sas à condition que l'hétérocycle ne puisse pas contenir 2 atomes contigus sélectionnés parmi O et S ; et où le groupe hétéroaryle dans le groupe hétéroaryle C₁-C₄-alkyle est un hétérocycle aromatique à 5 ou 6 membres, dans lequel les atomes membres de l'anneau de l'anneau hétérocycliques incluent, outre les atomes de carbone 1, 2, 3 ou 4 hétéroatomes sélectionnés parmi N, 0, et atomes membres de l'anneau Sas à condition que l'hétérocycle ne puisse pas contenir 2 atomes contigus sélectionnés parmi O et S ; et où l'un des groupes aliphatiques ou cycliques mentionnés ci-dessus est non substitué ouremplacée par 1, 2, 3, ou jusqu'au nombre maximal possible de groupes identiques ou différents R1a; ou
R¹ et R², ainsi que l'atome d'azote auquel ils sont attachés, forment un hétérocycle mono- ou bicyclique saturé ou partiellement insaturé de 3 à 10 membres, où l'hétérocycle inclut, à côté d'un atome d'azote et d'un ou plusieurs atomes de carbone, non d'autres hétératomes ou 1, 2 ou 3 hétératomes supplémentaires sélectionnés indépendamment parmi N, 0, et S comme atomes membres de l'anneau à condition que l'hétérocycle ne puisse pas contenir 2 atomes contigus sélectionnés parmi O et S ; et où l'hétérocycle est non substitué ou remplacé par 1, 2, 3, 4, ou jusqu'au nombre maximal possible de groupes identiques ou différents R1a ; où
R^{1a} est halogène, oxo, cyano, NO₂, OH, SH, NH₂, C₁-C₆-alkyle, C₁-C₆-haloalkyle, C₁-C₆-alkoxy, C₁-C₆-haloalkoxy, C₁-C₆-alkylthio, C₁-C₆-haloalkylthio, C₃-C₈-cycloalkyle, -NHSO₂- C₁-C₆-alkyle, -(C=O)- C₁-C₄-alkyle, C(=O)- C₁-C₄-alkyle, C₁-C₆-alkylsulfonyl, hydroxy C₁-C₄-alkyle, - C(=O)-NH2, -C(=O)-NH(C₁-C₄alkyl), C₁-C₄-alkylthio- C₁-C₄-alkyl, amino C₁-C₄-alkyl, C₁-C₄ alkylamino- C₁-C₄-alkyl, di C₁-C₄-alkylamino- C₁-C₄-alkyl, aminocarbonile- C₁-C₄-alkyl, ou C₁-C₄-alkoxy- C₁-C₄-al kyl ; R² est l'hydrogène, C₁-C₆-alkyle, C₂-C₆-alkyl, C₂-C₆-alkynyle, C₁-C₆-alkoxy, C₃-C₁₁ cycloalkyl, -C(=O)H, -C(=O)- C₁-C₆-alkyle, -C(=O)- C₃-C₁₁cycloalkyle, ou -C(=O)-O- C1-C6-alkyle ; et où n'importe lequel des groupes aliphatiques ou cycliques de R² est non substitué ou remplacé par 1, 2, 3, ou jusqu'au maximum possible de radicaux identiques ou différents sélectionnés parmi le groupe composé d'halogène, hydroxy, oxo, cyano, C₁-C₆-alkyle, C₁-C₆-alkoxy et C₃-C₁₁ cycloalkyle ;
pour obtenir un composé de formule IV

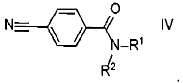

15. Le procédé, selon l'affirmation 14, comprend en outre la réaction du composé de la formule IV pour obtenir un composé de la formule V

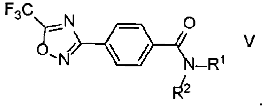

16. Le procédé, selon la revendication 15, comprend en outre l'étape de la réaction du composé de la formule V pour obtenir un composé de la formule VI

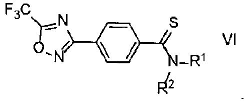

17. Le processus selon l'une des revendications 14 à 16, dans lequel dans les composés de formule III, IV, Vand VI
R¹ est méthyl ou 2-fluoro-phényle ; et
R² c'est de l'hydrogène.
